# EUROPÄISCHE PATENTANMELDUNG

(11) **EP 0 791 364 A2**
(43) Veröffentlichungstag der Anmeldung: **27.08.1997**
(21) Anmeldenummer: 97102743.8
(22) Anmeldetag: 20.02.1997
(51) Int. Cl.: A61L 9/01

(54) **Gemisch auf der Basis acyclischer Riechstoffe**

(30) Priorität: 24.02.1996 DE 29603379 U
(71) Anmelder: Schlaitzer Landwirtschaftliche Tierzucht GmbH, 06774 Schlaitz (DE); Stephan, Ursula, Prof. Dr., 06120 Halle/S. (DE); Strobel, Ute, Dr., 04155 Leipzig (DE); Kochmann, Werner, Prof. Dr., 06766 Wolfen (DE)
(72) Erfinder: Rösch, Werner, Dipl.-Landwirt, 06774 Schlaitz (DE); Stephan, Ursula, Prof. Dr., 06120 Halle/S (DE); Strobel, Ute, Dr., 04155 Leipzig (DE); Kochmann, Werner, Prof. Dr., 06766 Wolfen (DE)
(74) Vertreter: Tragsdorf, Bodo, Dipl.-Ing.

(57) **Zusammenfassung**

Die Erfindung betrifft ein Gemisch auf der Basis acyclischer Riechstoffe.
Ein Problem der Großtierhaltung besteht vor allem bei Schweinen oder Rindern in der in den Ställen herrschenden Geruchsbelästigung, die sich sowohl nachteilig auf das Arbeitspersonal als auch das Wohlbefinden der Tiere auswirkt, insbesondere deren Freßlust beeinträchtigt.
Ein weiteres Problem stellt die Geruchsbelästigung der anfallenden Gülle dar, vor allem wenn diese auf Wiesen aufgebracht wird, in deren unmittelbarer Nähe sich bewohnte Gebiete befinden.
Zur Lösung der genannten Probleme wird ein Gemisch folgender Zusammensetzung vorgeschlagen:
- 65 Ma-%: Citronellal
- 10 bis 15 Ma-%: Citronellol
- 3 bis 5 Ma-%: Geraniol
- 2 bis 3 Ma-%: Ameisensäure
- 1 bis 2 Ma-%: Essigsäure
- 2 bis 3 Ma-%: Citronellsäure
- 2 bis 3 Ma-%: Methylheptenon
und als Rest Ester und/oder Pinen.

Dieses wird entweder als Konzentrat oder mit Wasser verdünnt dem Güllesammelbecken zugegeben oder mittels Wasser versprüht oder mit einem Emulgatorgemisch versetzt.

## Beschreibung

Die Erfindung betrifft ein Gemisch auf der Basis acyclischer Riechstoffe der Monoterpenreihe.

Die bekannten Riechstoffe der Monoterpenreihe finden vor allem in der Parfümerie vielseitig Verwendung. Aus der EP 0 401 140 B1 ist eine desodorierende Zusammensetzung mit mindestens zwei Aldehyden und diese enthaltende desodorierende Produkte bekannt, zur Bekämpfung schlechter Gerüche. Als Aldehyde enthält diese Zusammensetzung u.a. auch Citronellal und Citral (Neral und Geraniol).
Aus der WO 87/07152 ist ein Mittel zur Bekämpfung des unangenehmen Geruchs der Exkremente von Haustieren bekannt. Dieses Mittel enthält als Zusammensetzung u.a. Citral und Linalool.
Aus der DE 43 40 609 A1 ist eine langsam verdampfende Zusammensetzung bekannt, deren Wirkstoff ein Terpentin ist, das Duft verbreitet, desinfizierend, insektizid und desodorierend oder Ozon-zersetzend wirkt. Außer dem Terpenoid enthält die Zusammensetzung noch ein Antioxidationsmittel und ein polymeres Netzwerkmaterial, auf dem das Antioxidationsmittel und das Terpenoid absorbiert sind. Als Terpenoide kommen u.a. Linalool, Citronellal und Citral in Frage.
Ein Problem der Großtierhaltung besteht vor allem bei Schweine- oder Rinderställen in der in den Ställen herrschenden Geruchsbelästigung, die sich sowohl auf das Verhalten des Arbeitspersonals und auch das Wohlbefinden der Tiere auswirkt. Der sich in den Ställen bildende Geruch entsteht sowohl durch die Exkremente als auch die Körperausdunstungen der Tiere. Außerdem bilden sich noch Gerüche durch das den Tieren bereitgestellte Futter, z.B. Silagefutter oder das typische Schweinefutter.
In Langzeitstudien wurde u.a. festgestellt, daß die Geruchskombination sich nachteilig auf die Freßlust der Tiere auswirkt und das Wohlbefinden der Tiere beeinträchtigt. So kommt es in Schweineställen häufig zum Schwanzbeißen der Tiere.
Ein weiteres Problem stellt die Geruchsbelästigung der anfallenden Gülle dar, vor allem wenn diese auf umliegende Wiesen aufgebracht werden kann und sich in unmittelbarer Nähe der Wiesen bewohnte Gebiete befinden. Die Bereitstellung entsprechender Mittel zur Bekämpfung der sich in den Ställen bildenden Gerüche ist letztlich auch eine Frage der Kosten.
Ein solches Mittel sollte nur geringe Kosten verursachen, nach Möglichkeit langanhaltende Wirkung haben mit gleichzeitiger Geruchsdämpfung für die anfallende Gülle und sich nicht nachteilig auf das Verhalten der Tiere auswirken.
Bisher ist kein Mittel bekannt, das geeignet ist diese Anforderungen zu erfüllen.

Der Erfindung lag die Aufgabe zugrunde, ein Mittel bereitzustellen, das die genannten Anforderungen erfüllt.

Erfindungsgemäß wird die Aufgabe durch die im Anspruch 1 angegebene Zusammensetzung gelöst. Weitere Ausgestaltungen sind in den Ansprüchen 2 bis 9 angegeben. Die Komponenten Citronellal, Citronellol und Geraniol können entweder aus den bekannten natürlichen Ölen Boromia citriodora Gum, dem Java-Citronellöl Cympobopogon Winterianus Jowill oder dem Palmarosaöl Cymbopogon Martini Stapf. oder dem Ceylon-Citronellöl Cymbopogon nardus Rendle gewonnen werden oder auf synthetischem Wege hergestellt werden. In Verbindung mit den weiteren Zusätzen an Ameisensäure, Essigsäure und Methylheptenon entsteht ein Gemisch mit einer Geruchs-Kombination, die nicht nur angenehm riecht, sondern vor allem bei Tieren das Geruchsempfinden derart beeinflußt, daß überlagerte unangenehme Gerüche, wie z.B. durch die Gülle hervorgerufen, nicht mehr wahrgenommen werden. Als Ester können Essigester, Ameisensäureester oder Citronellsäureester zugesetzt werden. In praktischen Versuchen in Schweineställen zeigte sich, daß dem Güllesammler zugesetzte geringe Mengen dieses Gemisches sich auf das Verhalten der Tiere sehr positiv auswirkte. Die Tiere verhielten sich wesentlich ruhiger und das ansonsten auffällige Schwanzbeißen der Tiere war nicht mehr festzustellen.
Besonders überraschend war, daß bei den Tieren nachweislich eine gesteigerte Freßlust festzustellen war, die zu einer deutlich schnelleren Gewichtszunahme führte. Die Geruchsbeeinflussung durch die Zugabe des erfindungsgemäßen Gemisches wurde von dem Arbeitspersonal auch als angenehm empfunden. Ein weiterer Vorteil ist die geringe erforderliche Einsatzmenge. Da es sich bei dem Gemisch generell um eine sehr kostengünstige Zusammensetzung handelt, entstehen bei einer langfristigen Stallbehandlung nur geringe Zusatzkosten. Das Gemisch kann entweder als Konzentrat mit Wasser verdünnt direkt dem Güllesammelbehälter zugegeben werden oder mittels Wasser versprüht werden. Die Applikationsmenge beträgt 15 g bis 25 g pro Liter Wasser. Ein weiterer Vorteil besteht auch darin, daß bei einer kontinuierlichen Behandlung der Ställe, z.B. 2 x pro Woche, der unangenehme Geruch der Gülle unterdrückt wird, und die Gülle nach dem Ausbringen ins Freie, z.B. auf Wiesen, auch für den Menschen keine Geruchsbelästigung mehr darstellt.
In Versuchen wurde auch festgestellt, daß das erfindungsgemäße Gemisch sehr gute bakteridzide Eigenschaften hat und insbesondere zur erfolgreichen Bekämpfung der Bakterien Enterococcus faecalis und Pseudomonas aeruginosa geeignet ist.
Das vorgeschlagene Gemisch kann auch als Bakterizid und Deodorant für Sammel- und Mobiltoiletten eingesetzt werden sowie zur Behandlung von Geruchsbelästigungen durch Klärschlamm, anaerobe Schlämme aus Fermentationsprozessen und Tierkörperreste.
Im Vergleich zu bekannten Mitteln zeichnet sich das neue Gemisch vor allem durch eine relativ lang anhaltende geruchshemmende und -überdeckende Wirkung aus. Dadurch kommt es zu einer wesentlichen Reduzierung der erforderlichen Einsatzmengen und die Behandlungskosten verringern sich somit zwangsläufig.

Die Erfindung soll nachstehend an einigen Beispielen erläutert werden. Es wurden Gemische folgender Zusammensetzung hergestellt.

| | Gemische (Angaben in g) | |
|---|---|---|
| | A | B |
| Citronellal | 70 | 78 |
| Citronellol | 15 | 10 |
| Geraniol | 5 | 3 |
| Ameisensäure | 3 | 2 |
| Essigsäure | 1 | 1 |
| Citronellsäure | 2 | 2 |
| Methylheptonon | 2 | 2 |
| Pinen | 1 | 2 |
| Essigester | 1 | - |

Außerdem wurden noch die Gemische A und B mit einem Emulgatorgemisch E aus Isotridecanolethyloxylat und Na-Ethylhexylsulfat zu jeweils gleichen Anteilen versetzt.
- Gemisch C:: 70 Masseanteile des Gemisches A und 30 Masseanteile des Gemisches E.
- Gemisch D:: 20 Masseanteile des Gemisches B und 80 Masseanteile des Gemisches E.

### Beispiel 1

Ein Aufzuchtstall für Ferkel mit einer Grundfläche von 30 m x 6 m für 400 Ferkel wurde mit dem Gemisch A behandelt. Das Alter der Tiere betrug 35 Tage. Die Raumtemperatur im Aufzuchtstall lag zwischen 20 ^{o}C und 28 ^{o}C. Den Ferkeln wurde das übliche Ferkelfutter E 25 verabreicht. In dem Gülleauffangbecken unterhalb der Boxen wurde während einer Zeitdauer von 45 Tagen 2 x in der Woche eine Menge von 50 g des Gemisches A, verdünnt mit 2,5 l Wasser, in die Gülleauffangbecken gegeben.
Zu Beginn der Versuchsreihe hatten die Ferkel ein durchschnittliches Gewicht von 8,5 kg. Nach Abschluß der Versuchsreihe nach 45 Tagen lag das durchschnittliche Gewicht der Jungschweine bei 28 kg. Die bereitgestellte Futtermenge betrug im Tagesdurchschnitt 1,2 kg pro Tier.

### Beispiel 2

In einem Maststall für Jungschweine im Alter von 80 Tagen mit einem Tierbestand von 300 Schweinen wurden die Gülleauffangbecken unterhalb der Boxen mit dem Gemisch B behandelt, während einer Zeitdauer von 120 Tagen. Der Maststall hat eine Grundfläche von 45 m x 7,5 m und die Temperatur im Stall liegt zwischen 20 ^{o}C und 28 ^{o}C. Die Schweine wurden mit Aufzuchtfutter in ausreichender Menge gefüttert. Das Gemisch B wurde 2 x pro Woche in einer Menge von 40 g, verdünnt mit 2,5 l Wasser in die Gülleauffangbecken gegeben. Zu Beginn der Versuchsreihe hatten die Tiere ein durchschnittliches Gewicht von 28 kg. Nach 120 Tagen betrug das durchschnittliche Gewicht der Tiere 103 kg . Die im Versuchszeitraum bereitgestellte tägliche Futtermenge pro Tier betrug am 1. Tag 1,2 kg und wurde bis zum 120. Tag auf 2,5 kg erhöht.

### Veraleichsbeispiel 1

Unter den gleichen Bedingungen wie im Beispiel 1 jedoch ohne Zugabe des Gemisches A, wurden Ferkel aufgezogen.
Das durchschnittliche Gewicht der Ferkel im Alter von 35 Tagen betrug 8, 5 kg. Nach 45 Tagen erreichten die Jungschweine ein durchschnittliches Gewicht von 25 kg.

### Vergleichsbeispiel 2

Unter den gleichen Bedingungen wie in Beispiel 2, jedoch ohne Zugabe des Gemisches B, wurden Jungschweine aufgezogen.
Das durchschnittliche Gewicht der Jungschweine betrug 28 kg. Nach einer Zeitdauer von 120 Tagen wurde ein durchschnittliches Gewicht von 95 kg je Jungschwein erreicht.

Wie die Beispiele zeigen, kann durch die Zugabe des erfindungsgemäßen Gemisches zu der Gülle in den Ställen bzw. durch Versprühen eine Beruhigung im Verhalten der Tiere festgestellt werden, die sich in einer gesteigerten Freßlust zeigt. In Versuchsreihen konnten zusätzliche Gewichtszunahmen von ca. 10 % ermittelt werden.

### Beispiel 3

Nach den Vorschriften der Deutschen Gesellschaft für Hygiene und Mikrobiologie (DGHM) wurde im qualitativen Suspensionsversuch die bakterizide Wirkung der Gemische A, B, C und D untersucht. Die jeweiligen Gemische wurden in verschiedenen Mengenanteilen von 2 %, 1 %, 0,5 % und 0,25 % dem Bakterienstamm Enterococcus faecalis und dem Stamm Pseudomonas aeruginosa zugegeben. Nach einer Zeitdauer von 3 Tagen wurde festgestellt, daß bei allen Versuchen die Mikroben vollständig abgetötet waren.

### Beispiel 4

In einem Laborversuch wurde ein Gemisch aus 120 g frischen Fäkalien und 825 ml frischem Urin hergestellt, wobei die Mischungsbestandteile innig miteinander in einem Laborglas verrührt wurden. Danach wurden jeweils 50 ml dieses Gemisches mit 50 ml eines Wasser-Wirkstoff-Gemisches unterschiedlicher Konzentrationen in einem Rührgefäß mit einem Propellerrührer verrührt (Rührgeschwindigkeit 500 bis 1000 U/min und Rührdauer 5 min). Die Konzentrationen der Gemische A, B, C und D betrugen jeweils 5, 2, 1, 0,5 und 0,1 %. Die einzelnen Proben wurden jeweils nach einer Zeitdauer von 8, 24, 48 und 96 Stunden hinsichtlich der Geruchsbildung untersucht, wobei als Vergleichsprobe eine Probe diente, die anstelle des Wirkstoffgemisches Wasser enthielt und in der nachfolgenden Tabelle mit V gekennzeichnet wurde.
Die Bewertung führte zu folgenden Ergebnissen:

| Gemisch | Konzentration in % | | | | |
|---|---|---|---|---|---|
| | 5 | 2 | 1 | 0.5 | 0.1 |
| V | 4 | 4 | 4 | 4 | 4 |
| A | 0 | 0 | 1 | 1 | 2 |
| B | 0 | 1 | 1 | 1 | 2 |
| C | 0 | 0 | 0 | 0 | 1 |
| D | 0 | 0 | 0 | 0 | 1 |

Die Zahlenangaben in der vorstehenden Tabelle haben folgende Bedeutung:
- 4: sehr starke Geruchsbelästigung (= keine Wirkung)
- 3: starke Geruchsbelästigung (= sehr geringe Wirkung)
- 2: moderate Geruchsbelästigung (= Geruch des Wirkstoffgemisches ist spürbar)
- 1: geringe Geruchsbelästigung (= Geruch des Wirkstoffgemisches ist deutlich bemerkbar)
- 0: keine Geruchsbelästigung (= Geruch des Wirkstoffgemisches ist dominierend)

### Beispiel 5

In geleerte und gereinigte Mobiltoiletten wurden 20 l Wasser gegeben und diese jeweils mit 20ml, 50 ml, 100 ml und 200 ml der Wirkstoffgemische A, B, C und D versetzt. Das maximale Füllstandsvolumen der Toiletten beträgt 320 l. Die Toiletten wurden bei Außentemperaturen von 22 bis 28 ^{o}C an normal frequentierten Orten aufgestellt. Für jede Versuchsreihe wurden zehn Toiletten eingesetzt. Nach einer Zeitdauer von 7 Tagen wurde die Geruchsbelästigung der Toiletten bewertet.
Die ermittelten Ergebnisse sind in der nachfolgenden Tabelle angegeben:

| Gemisch | Konzentration in ml | | | |
|---|---|---|---|---|
| | 20 | 50 | 100 | 200 |
| A | 3,1 | 1,8 | 0,2 | 0 |
| B | 2,8 | 1,5 | 0,3 | 0 |
| C | 1,8 | 1,2 | 0,1 | 0 |
| D | 1,5 | 0,9 | 0 | 0 |

Die in der Tabelle angegebenen Zahlenangaben haben die gleiche Bedeutung wie in Beispiel 3 und stellen die Mittelwerte von jeweils 10 Versuchsreihen dar.

### Beispiel 6

An technischen Abfallprodukten mit einer starken Geruchsbelästigung wurde die desodorierende Wirkung der Gemische A, B, C und D untersucht.

### Abfallprodukte:

- I: frischer Klärschlamm aus einer biologischen Abwasseranlage
- II: Tierkörperreste aus einer Abdeckerei
- III: anaerober Schlamm aus einer Fermentationsanlage eines Pharmabetriebes

In Laborversuchen werden die Abfallprodukte I, II und III in einer Schichtdicke von 2 cm auf eine Glasplatte mit einer Grundfläche von 50 x 50 cm aufgetragen und mit den Wirkstoffgemischen A, B, C und D in unterschiedlichen Konzentrationen besprüht, wobei die Verdünnung mit Wasser erfolgte. Die versprühte Menge betrug jeweils 1 ml/cm². Nach einer Zeitdauer von 1, 4, 8 und 24 Stunden wurden die einzelnen Proben hinsichtlich der Geruchsbelästigung bewertet. Die ermittelten Ergebnisse sind in der nachfolgenden Tabelle angegeben. Die Zahlen 0 bis 4 entsprechen der im Beispiel 3 angegebenen Bedeutung.

| Abfallprodukt I: | | | | | | |
|---|---|---|---|---|---|---|
| Gemisch | Bewertungszeit nach Stunden | Konzentration in % | | | | |
| | | 100 | 50 | 10 | 5 | 2 |
| A | 1 | 0 | 0 | 0 | 1 | 2 |
| | 4 | 0 | 0 | 1 | 2 | 2 |
| | 8 | 0 | 0 | 1 | 2 | 3 |
| | 24 | 1 | 1 | 2 | 2 | 3 |
| B | 1 | 0 | 0 | 0 | 1 | 2 |
| | 4 | 0 | 1 | 1 | 2 | 3 |
| | 8 | 0 | 0 | 1 | 2 | 3 |
| | 24 | 1 | 0 | 2 | 2 | 3 |
| C | 1 | 0 | 0 | 0 | 1 | 2 |
| | 4 | 1 | 0 | 1 | 2 | 2 |
| | 8 | 1 | 1 | 1 | 2 | 3 |
| | 24 | 0 | 1 | 1 | 2 | 3 |
| D | 1 | 0 | 0 | 1 | 1 | 2 |
| | 4 | 0 | 1 | 1 | 2 | 2 |
| | 8 | 1 | 0 | 1 | 2 | 3 |
| | 24 | 0 | 1 | 1 | 3 | 3 |

| Abfallprodukt II: | | | | | |
|---|---|---|---|---|---|
| Gemisch | Bewertungszeit nach Stunden | Konzentration in % | | | |
| | | 100 | 50 | 10 | 5 |
| A | 1 | 1 | 2 | 2 | 3 |
| | 4 | 2 | 2 | 3 | 4 |
| | 8 | 2 | 2 | 4 | 4 |
| | 24 | 3 | 3 | 4 | 4 |
| B | 1 | 1 | 2 | 3 | 3 |
| | 4 | 2 | 2 | 3 | 4 |
| | 8 | 2 | 2 | 4 | 4 |
| | 24 | 2 | 3 | 4 | 4 |
| C | 1 | 2 | 2 | 3 | 4 |
| | 4 | 2 | 3 | 4 | 4 |
| | 8 | 3 | 3 | 4 | 4 |
| | 24 | 4 | 4 | 4 | 4 |
| D | 1 | 2 | 2 | 4 | 4 |
| | 4 | 2 | 3 | 4 | 4 |
| | 8 | 3 | 4 | 4 | 4 |
| | 24 | 4 | 4 | 4 | 4 |

| Abfallprodukt III: | | | | | | |
|---|---|---|---|---|---|---|
| Gemisch | Bewertungszeit nach Stunden | Konzentration in % | | | | |
| | | 100 | 50 | 10 | 5 | 2 |
| A | 1 | 0 | 0 | 1 | 1 | 2 |
| | 4 | 1 | 1 | 2 | 2 | 2 |
| | 8 | 1 | 1 | 2 | 3 | 3 |
| | 24 | 1 | 1 | 3 | 4 | 4 |
| B | 1 | 0 | 0 | 1 | 1 | 2 |
| | 4 | 1 | 1 | 2 | 2 | 3 |
| | 8 | 1 | 1 | 2 | 3 | 3 |
| | 24 | 2 | 2 | 2 | 3 | 4 |
| C | 1 | 0 | 1 | 1 | 2 | 3 |
| | 4 | 1 | 1 | 2 | 3 | 4 |
| | 8 | 1 | 2 | 3 | 3 | 4 |
| | 24 | 2 | 2 | 3 | 4 | 4 |
| D | 1 | 1 | 1 | 1 | 2 | 3 |
| | 4 | 1 | 1 | 2 | 3 | 4 |
| | 8 | 1 | 2 | 3 | 4 | 4 |
| | 24 | 2 | 2 | 3 | 4 | 4 |

## Patentansprüche

1. Gemisch auf der Basis acyclischer Riechstoffe der Monoterpenreihe, bestehend aus
mindestens
65 Ma-% Citronellal
10 bis 15 Ma-% Citronellol
3 bis 5 Ma-% Geraniol
2 bis 3 Ma-% Ameisensäure
1 bis 2 Ma-% Essigsäure
2 bis 3 Ma-% Citronellsäure
2 bis 3 Ma-% Methylheptenon
und als Rest Ester und/oder Pinen.

2. Gemisch nach Anspruch 1, dadurch gekennzeichnet, daß der Ester Ameisensäureester, Essigester oder Citronellsäureester ist.

3. Gemisch nach einem der Ansprüche 1 oder 2, dadurch gekennzeichnet, daß dieses mit Wasser in einem Mengenanteil von 15 g/l bis 25 g/l Wasser verdünnt ist.

4. Gemisch nach einem der Ansprüche 1 bis 3, dadurch gekennzeichnet, daß dieses mit einem Emulgatorgemisch, bestehend aus biologisch abbaubaren nichtionogenen und anionenaktiven Tensiden zu gleichen Mengenanteilen, versetzt ist, wobei die Mengenanteile des Gemisches 20 bis 70 % und die Mengenanteile des Emulgatorgemisches 30 bis 80 % betragen.

5. Verwendung eines Gemisches nach einem der Ansprüche 1 bis 3 zur Stimulierung des Wohlbefindens und der Freßlust von in Ställen untergebrachten Tieren sowie zur Geruchsunterdrückung der Gülle.

6. Verwendung eines Gemisches nach einem der Ansprüche 1 bis 4 als Bakterizid.

7. Verwendung eines Gemisches nach einem der Ansprüche 1 bis 4 zur Bekämpfung der Bakterien Enterococcus faecalis und/oder Pseudomonas aeruginosa.

8. Verwendung eines Gemisches nach einem der Ansprüche 1 bis 4 als Bakterizid und Deodorant für Sammel- und Mobiltoiletten.

9. Verwendung eines Gemisches nach einem der Ansprüche 1 bis 4 zur Behandlung von Geruchsbelästigungen durch Klärschlamm, anaeroben Schlämmen aus Fermentationsprozessen und Tierkörperreste.
